(19) Europäisches Patentamt
European Patent Office
Office européen des brevets

(11) EP 1 036 558 B1

(12) FASCICULE DE BREVET EUROPEEN

(45) Date de publication et mention de la délivrance du brevet: **04.09.2002 Bulletin 2002/36**

(51) Int Cl.[7]: **A61K 7/135**

(21) Numéro de dépôt: **00400150.9**

(22) Date de dépôt: **20.01.2000**

# (54) Composition anhydre de décoloration de fibres kératiniques comprenant l'association d'un polymère épaississant hydrosoluble et d'un polymère amphiphile non ionique comportant au moins une chaîne grasse

Wasserfreie Zusammensetzung zum Bleichen von keratinischen Fasern enthaltend eine Kombination von einem wasserlöslichen verdickenden Polymer und einem amphiphilischen nichtionischen Polymer enthaltend eine Fettkette

Anhydrous composition for decoloration of ceratinic fibers comprising a combination of a hydrosoluble thickening polymer and an amphiphilic nonionic polymer carrying a fatty chain

(84) Etats contractants désignés:
**AT BE CH CY DE DK ES FI FR GB GR IE IT LI LU MC NL PT SE**

(30) Priorité: **29.01.1999 FR 9901056**

(43) Date de publication de la demande:
**20.09.2000 Bulletin 2000/38**

(73) Titulaire: **L'OREAL**
**75008 Paris (FR)**

(72) Inventeurs:
- **Legrand, Frédéric**
**92100 Boulogne-Billancourt (FR)**
- **Millequant, Jean**
**94100 Saint-Maur (FR)**

(74) Mandataire: **Casalonga, Axel**
**BUREAU D.A. CASALONGA - JOSSE**
**Paul-Heyse-Strasse 33**
**80336 München (DE)**

(56) Documents cités:
**EP-A- 0 650 719**
**EP-A- 0 778 020**
**EP-A- 0 827 738**
**WO-A-92/03120**
**WO-A-98/03150**
**FR-A- 2 769 221**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention concerne des compositions anhydres pour la décoloration de fibres kératiniques comprenant l'association d'au moins un polymère amphiphile non ionique comportant au moins une chaîne grasse et d'au moins un polymère épaississant hydrosoluble, l'utilisation de ces compositions pour la préparation de compositions de décoloration prêtes-à-l'emploi, un procédé de décoloration de fibres kératiniques utilisant ces compositions, ainsi qu'un kit d'emballage contenant une telle composition.

Pour décolorer les cheveux, on utilise généralement des poudres décolorantes contenant un réactif peroxygéné tel que les persulfates, perborates ou percarbonates d'ammonium ou de métaux alcalins, que l'on associe au moment de l'emploi à une composition aqueuse de peroxyde d'hydrogène. Les sels peroxygénés et le peroxyde d'hydrogène étant relativement stables en milieu acide, il est nécessaire de les activer à pH basique pour obtenir une formation adéquate d'oxygène. Il est donc usuel d'ajouter aux poudres décolorantes des composés alcalins tels que les amines et les silicates alcalins.

Dans le domaine de la décoloration capillaire, on recherche généralement des compositions décolorantes suffisamment épaisses pour permettre une application précise sur certaines zones de la chevelure, et qui ne risquent pas de couler sur le visage ou en dehors des zones que l'on se propose de décolorer.

L'effet épaississant ou gélifiant est classiquement obtenu avec des épaississants traditionnels tels que les dérivés de cellulose, les dérivés d'amidon, les alginates ou les silices épaississantes.

Lorsqu'on utilise ces épaississants traditionnels, on constate cependant une importante diminution de la viscosité de la composition décolorante finale au cours du temps.

Il existe donc un besoin d'un système épaississant capable d'assurer le maintien d'une viscosité élevée pendant la durée nécessaire pour obtenir la décoloration souhaitée, généralement comprise entre dix minutes et une heure.

La demanderesse a fait la découverte surprenante qu'il était possible d'améliorer considérablement le maintien de la viscosité au cours du temps de compositions de décoloration, en associant aux épaississants hydrosolubles classiques, un polymère amphiphile non ionique comportant au moins une chaîne grasse.

On a également constaté qu'un tel système épaississant permettait des dilutions, avec des compositions aqueuses de peroxyde d'hydrogène, nettement plus importantes que les systèmes épaississants connus.

La présente invention a par conséquent pour objet une composition anhydre de décoloration de fibres kératiniques, en particulier humaines, comprenant, dans un milieu approprié pour la décoloration, au moins un agent alcalin, au moins un sel peroxygéné, et en outre l'association

- d'au moins un polymère épaississant hydrosoluble, et
- d'au moins un polymère amphiphile non ionique comportant au moins une chaîne grasse.

L'invention a également pour objet l'utilisation d'une telle composition pour la préparation d'une composition de décoloration prête-à-l'emploi.

Elle a en outre pour objet un procédé de décoloration des fibres kératiniques utilisant la composition de décoloration anhydre décrite ci-dessus, ainsi qu'un kit d'emballage contenant une telle composition.

D'autres objets apparaîtront à la lecture de la description et des exemples qui suivront.

Les polymères épaississants hydrosolubles utilisables selon la présente invention englobent tous les polymères hydrosolubles synthétiques ou d'origine naturelle utilisés classiquement dans le domaine cosmétique.

On peut citer comme exemples de polymères épaississants synthétiques la polyvinylpyrrolidone, l'acide polyacrylique, le polyacrylamide, l'acide polyacrylamidométhylpropanesulfonique ou des copolymères de ceux-ci, ces polymères étant réticulés ou non réticulés.

Les polymères épaississants d'origine naturelle utilisables selon la présente invention sont des polymères comportant au moins un motif de sucre, à savoir

(a) les gommes de guar non-ioniques ;
(b) les gommes de biopolysaccharides d'origine microbienne telles que les gommes de scléroglucane et de xanthane ;
(c) les gommes issues d'exsudats végétaux telles que la gomme arabique, la gomme ghatti, la gomme karaya ou la gomme adragante ;
(d) les gommes extraites d'algues telles que les carraghénanes ou l'agar,
(e) les gommes issues d'extraits végétaux tels que la gomme de caroube ou les pectines extraites de pulpes de fruits ;
(f) les alginates ;
(g) les amidons ; et
(h) les hydroxyalkylcelluloses et carboxyalkylcelluloses ;

On entend par le terme "motif de sucre" dans la présente invention une portion monosaccharidique ou une portion oligo- ou polysaccharidique constituée d'un même type de motifs saccharidiques (oligo- ou poly*holo*sides) ou de plusieurs types de motifs saccharidiques différents (oligo- ou poly*hétéro*sides).

Les unités saccharidiques de tous ces polymères peuvent porter un ou plusieurs substituants, par exemple des groupements alkyle, hydroxyalkyle, alcoxy, acyloxy ou carboxyle, les radicaux alkyle comportant de 1 à 4 atomes de carbone.

Les gommes de guar non ioniques peuvent être modifiées ou non modifiées. Les gommes de guar non modifiées sont par exemple des produits vendus sous la dénomination VIDOGUM GH 175 par la société UNI-PECTINE et sous la dénomination JAGUAR C par la société MAYHALL.

Selon la présente invention, on peut aussi utiliser des gommes de guar non ioniques modifiées par des groupements hydroxyalkyle en $C_{1-4}$, par exemple hydroxyméthyle, hydroxyéthyle, hydroxypropyle et hydroxybutyle.

Ces gommes de guar modifiées sont bien connues dans la technique et peuvent être préparées par réaction de la gomme de guar avec les oxydes d'alkylène appropriés. Le taux d'hydroxyalkylation (rapport du nombre de molécules d'oxyde d'alkylène fixées au nombre initial de groupes hydroxyle libres) est de préférence compris entre 0,4 et 1,2.

De telles gommes de guar non ioniques modifiées sont par exemple vendues sous les dénominations JAGUAR HP8, JAGUAR HP60, JAGUAR HP120, JAGUAR DC293 et JAGUAR HP105 par la société RHONE POULENC (MAYHALL) ou sous la dénomination GALACTA-SOL 4H4FD2 par la société AQUALON.

Les gommes de biopolysaccharides d'origine microbienne telles que les gommes de scléroglucane ou de xanthane, les gommes issues d'exsudats végétaux telles que la gomme arabique, la gomme ghatti, la gomme karaya, la gomme adragante, les extraits d'algues tels que les carraghénanes ou l'agar, les extraits végétaux tels que la gomme de caroube ou les pectines, les alginates, les amidons, les hydroxyalkylcelluloses et carboxyalkylcelluloses sont bien connus de l'homme du métier et sont décrits notamment dans "Handbook of Water Soluble Gums and Resins" de Robert L. Davidson, édité chez Mc Graw Hill Book Company (1980).

Parmi ces gommes, les scléroglucanes sont représentés par les produits vendus par la société SANOFI BIO INDUSTRIES sous la dénomination ACTIGUM CS, et en particulier sous la dénomination ACTIGUM CS 11, et par la société ALBAN MULLER INTERNATIONAL sous la dénomination AMIGEL.

On peut également utiliser d'autres scléroglucanes, par exemple un scléroglucane traité au glyoxal décrit dans la demande de brevet FR-A-2 633 940.

Les gommes de xanthane utilisables en tant qu'épaississants dans les compositions de la présente invention sont par exemple représentées par les produits vendus sous les dénominations KELTROL, KEL-TROLT, KELTROLTF, KELTROLBT, KELTROL RD et KELTROL CG par la société NUTRASWEET KELCO, ou sous les dénominations RHODICARE S ou RHODICARE H par la société RHODIA CHIMIE.

Les hydroxyalkylcelluloses sont généralement des hydroxy(alkyl en $C_{1-4}$)-celluloses et plus particulièrement des hydroxyéthylcelluloses. Elles sont disponibles par exemple sous les dénominations CELLOSIZE QP3L, CELLOSIZE QP4400H, CELLOSIZE QP30000H, CELLOSIZE HEC30000A ou CELLOSIZE POLYMER PCG10 par la société AMERCHOL, sous les dénominations NATROSOL 250HHR, NATROSOL 250MR, NATROSOL 250M, NATROSOL 250HHXR, NATROSOL 250HHX, NATROSOL 250HR, NATROSOL HX par la société HERCULES ou encore sous la dénomination TYLOSE H1000 par la société HOECHST.

Les hydroxyalkylcelluloses peuvent également être des hydroxypropylcelluloses vendues sous les dénominations KLUCEL EF, KLUCEL H, KLUCEL LHF, KLUCEL MF, KLUCEL G par la société AQUALON.

Parmi les carboxyalkylcelluloses, on utilise de préférence la carboxyméthylcellulose qui est commercialisée par exemple sous les dénominations BLANOSE 7M8/SF, BLANOSE RAFFINÉE 7M, BLA-NOSE 7LF, BLANOSE 7MF, BLANOSE 9M31F, BLANOSE 12M31XP, BLANOSE 12M31P, BLANOSE 9M31XF, BLANOSE 7H, BLANOSE 7M31, BLANOSE 7H3SXF par la société AQUALON, ou sous les dénominations AQUASORB A500 et AMBERGUM 1221 par la société HERCULES, sous les dénominations CELLOGEN HP810A et CELLOGEN HP6HS9 par la société MONTELLO, ou encore sous la dénomination PRI-MELLOSE par la société AVEBE.

Les polymères épaississants hydrosolubles particulièrement préférés utilisables en tant qu'agents épaississants classiques dans la composition décolorante anhydre de la présente invention sont les gommes de guar, les dérivés de gomme de guar ou les hydroxyalkylcelluloses.

Le ou les épaississant(s) hydrosoluble(s) décrit(s) ci-dessus sont utilisés généralement à raison de 0,03 à 30 % en poids, de préférence à raison de 0,3 à 15 % en poids, par rapport à la composition anhydre.

Pour obtenir les propriétés rhéologiques intéressantes indiquées ci-avant, c'est-à-dire une viscosité élevée et stable au cours du temps, même pour des dilutions importantes, il est nécessaire selon la présente invention, d'adjoindre aux polymères épaississants hydrosolubles décrits ci-dessus, des polymères amphiphiles non ioniques comportant au moins une chaîne grasse.

Les polymères amphiphiles non ioniques comportant au moins une chaîne grasse utilisables selon la présente invention englobent par exemple :

- les celluloses ou hydroxyalkylcelluloses modifiées par des groupements comportant au moins une chaîne grasse telle qu'un groupe alkyle, arylalkyle ou alkylaryle contenant un groupe alkyle de préférence en $C_{8-22}$ comme les produits NATROSOL PLUS GRADE 330 CS de la société AQUALON, BERMOCOLL EHM 100 de la société BEROL NOBEL, ou POLYSURF 67 de la société HERCULES), ou modifiées par des groupes alkylphénol polyalcoxylés comme le produit AMERCELL POLYMER HM-1500 de la société AMERCHOL;
- les hydroxypropylguars modifiés par des groupements comportant au moins une chaîne grasse en $C_{8-22}$ comme les produits ESAFLOR HM 22 (chaîne alkyle en $C_{22}$) de la société LAMBERTI, MIRACARE XC95-3 (chaîne alkyle en $C_{14}$) et RE205-1 (chaîne alkyle en $C_{20}$) de la société RHONE POULENC;
- les polyuréthannes comportant au moins une chaîne grasse de type alkyle ou alcényle en $C_{8-30}$ comme le SER-AD FX 1100 de la société SERVO DELBEN,
- le copolymère SMDI (Saturated Methylene Diphenyl Diisocyanate) polyéthylèneglycol(s) avec terminaison décyle,
- le copolymère SMDI (Saturated Methylene Diphenyl Diisocyanate) polyéthylène glycol(s) avec terminaison alkyle (méthyle/$C_{18}$), associé à une matrice maltodextrine, ou
- le diuréthane HMDI (Hexa Methylene Di Isocyanate) d'alcools en $C_{10-18}$ oxyéthylénés (66 OE) et oxypropylénés (14 OP) commercialisé sous la dénomination ELFACOS T 212 par la société AKZO,
- les copolymères de vinylpyrrolidone et de monomères hydrophobes à chaîne grasse comme les produits ANTARON V216 ou GANEX V216 (poly(vinylpyrrolidone/hexadécène)), ANTARON V220 ou GANEX V220 (poly(vinylpyrrolidone/eicosène)) de la société I.S.P. ;
- les copolymères de (méth)acrylates d'alkyle en $C_{1-6}$ et de monomères amphiphiles comportant au moins une chaîne grasse ;
- les copolymères de (méth)acrylates hydrophiles et de monomères hydrophobes comportant au moins une chaîne grasse, par exemple un poly(méthacrylate de polyéthylèneglycol/méthacrylate de lauryle).

On préfère en particulier les polyuréthannes comportant au moins une chaîne grasse de type alkyle en $C_{10-20}$ et les hydroxyéthylcelluloses modifiées par des groupements comportant au moins un radical alkyle en $C_{8-22}$.

Ces polymères amphiphiles non ioniques sont utilisés à raison de 0,03 à 30 % en poids, de préférence à raison de 0,3 à 15 % en poids, par rapport à la composition décolorante anhydre.

Le rapport en poids du polymère amphiphile non ionique comportant au moins une chaîne grasse au polymère épaississant hydrosoluble est généralement compris entre 10/1 et 1/10 et de préférence entre 5/1 et 1/5.

La composition décolorante anhydre de la présente invention peut contenir, en plus du système épaississant constitué par au moins un polymère épaississant hydrosoluble et au moins un polymère amphiphile non ionique comportant au moins une chaîne grasse, au moins un polymère amphiphile anionique comportant au moins une chaîne grasse.

Ces polymères amphiphiles anioniques lorsqu'ils sont présents, jouent également le rôle d'agents épaississants et peuvent renforcer l'effet du système épaississant décrit ci-dessus.

Il s'agit le plus souvent de copolymères synthétiques, réticulés ou non réticulés, comprenant

- des motifs *hydrophiles* dérivés d'un ou de plusieurs monomères à insaturation éthylénique portant une fonction d'acide carboxylique libre, et
- des motifs *hydrophobes* dérivés d'un ou de plusieurs monomères à insaturation éthylénique portant une chaîne latérale hydrophobe, et éventuellement
- des motifs de réticulation dérivés d'un ou de plusieurs monomères polyinsaturés.

Le ou les monomères à insaturation éthylénique portant une fonction d'acide carboxylique sont choisis parmi l'acide éthacrylique, l'acide méthacrylique et l'acide acrylique, de préférence parmi l'acide méthacrylique, l'acide acrylique et des mélanges de ceux-ci.

Le ou les monomères à insaturation éthylénique portant une chaîne latérale hydrophobe peuvent être (i) des esters d'acides carboxyliques insaturés et d'alcools gras, ou (ii) des éthers d'allyle et d'alcools gras.

(i) Les esters d'acides carboxyliques insaturés et d'alcools gras sont choisis par exemple parmi les éthacrylates, méthacrylates et/ou acrylates d'alkyle en $C_{10-30}$, de préférence en $C_{12-22}$.

Ils englobent par exemple l'acrylate de lauryle, l'acrylate de stéaryle, l'acrylate de décyle, l'acrylate d'isodécyle, l'acrylate de dodécyle ainsi que les méthacrylates correspondants, à savoir le méthacrylate de lauryle, le méthacrylate de stéaryle, le méthacrylate de décyle, le méthacrylate d'isodécyle et le méthacrylate de dodécyle.

(ii) Les éthers allyliques d'alcools gras formant les motifs hydrophobes des polymères amphiphiles anioniques de la présente invention correspondent à la formule

(I) $CH_2=CR'CH_2-O-B_n-R$

dans laquelle

R' représente un atome d'hydrogène ou un groupe méthyle,
B représente un groupe éthylèneoxy,
n est un nombre entier valant entre 0 et 100,
R représente un groupe hydrocarboné choisi parmi les restes alkyle, arylalkyle, aryle, alkylaryle ou cycloalkyle comportant de 8 à 30 atomes de carbone, de préférence de 10 à 24 atomes de carbone, et plus particulièrement de 12 à 18 atomes de carbone.

Un motif de formule (I) préféré selon la présente invention est un motif dans lequel R' désigne un atome d'hydrogène, n est égal à 10 et R représente un radical stéaryle ($C_{18}$).

Ledit monomère réticulant est un composé comportant au moins deux doubles liaisons polymérisables non conjuguées l'une avec l'autre. On peut citer à titre d'exemple le phtalate de diallyle, le (méth)acrylate d'allyle, le divinylbenzène, le diméthacrylate de (poly)éthylèneglycol, le méthylène-bis-acrylamide, le polyallylsucrose ou le polyallylpentaérythritol.

Des polymères amphiphiles anioniques du type décrit ci-dessus sont décrits par exemple dans les brevets US-3 915 921 et US-4 509 949 (copolymères d'acide (éth/méth)acrylique et de (éth/méth)acrylates d'alkyle en $C_{10-30}$), ou dans le brevet EP-0 216 479 B2 (copolymères d'acide (éth/méth)acrylique et d'éthers allyliques d'alcools gras).

On peut citer à titre d'exemples de polymères préférés :

- les polymères réticulés d'acide acrylique et d'acrylate d'alkyle en $C_{10-30}$, tels que les polymères commercialisés sous les dénominations PEMULEN TR1, PEMULEN TR2 et CARBOPOL 1382 par la société GOODRICH,
- le polymère réticulé d'acide acrylique et de méthacrylate d'alkyle en $C_{10-30}$, tel que le CARBOPOL ETD 2020 commercialisé par la société GOODRICH,
- le terpolymère acide méthacrylique/acrylate d'éthyle/méthacrylate de stéaryle oxyéthyléné (55/35/10),
- le terpolymère acide (méth)acrylique/acrylate d'éthyle/méthacrylate de béhényle oxyéthyléné (25 OE), et
- le terpolymère acide méthacrylique/acrylate d'éthyle/stéareth-10 allyléther réticulé.

Ces polymères amphiphiles anioniques, sont présents, le cas échéant, dans les compositions décolorantes anhydres de l'invention à raison de 0,03 à 30 % du poids total de la composition.

Comme indiqué ci-dessus, la composition décolorante anhydre contient au moins un agent alcalin et au moins un sel peroxygéné.

Ledit agent alcalin est choisi parmi les silicates, phosphates ou carbonates de métaux alcalins ou alcalino-terreux, en particulier les métasilicates de métaux alcalins.

Les sels peroxygénés sont choisis parmi les persulfates, les percarbonates et les perborates d'ammonium ou de métaux alcalins.

On utilise de préférence les persulfates et parmi ceux-ci principalement les persulfates de sodium et de potassium.

Les compositions de l'invention comprennent entre 20 et 70 % en poids et de préférence entre 30 et 60 % en poids de sel(s) peroxygéné(s) par rapport au poids total de la composition anhydre.

Les compositions de décoloration anhydres selon la présente invention peuvent également contenir toutes sortes d'adjuvants de décoloration susceptibles de faciliter la manipulation et l'application, d'améliorer la conservation ou l'efficacité des compositions et d'améliorer les propriétés cosmétiques des cheveux traités.

Ces adjuvants sont par exemple des agents de contrôle du dégagement d'oxygène tels que le carbonate de magnésium et la magnésie, des agents tensioactifs anioniques, non ioniques, cationiques, amphotères ou zwitterioniques et leurs mélanges, des huiles minérales ou végétales, des cires, des adjuvants de granulation, des liants, des charges minérales telles que la silice et l'argile, des opacifiants tels que l'oxyde de titane, des colorants, des séquestrants, des parfums, et des polymères substantifs cationiques ou amphothères.

Bien entendu, l'homme de métier veillera à choisir ce (ou ces) éventuel(s) composé(s) supplémentaire(s) et sa (ou leur) quantité de manière à ce que les propriétés avantageuses attachées intrinsèquement à la composition de décoloration conforme à l'invention ne soient pas, ou substantiellement pas, altérées par la (ou les) adjonction(s) envisagée(s).

Les polymères substantifs cationiques utilisables conformément à la présente invention peuvent être choisis parmi tous ceux déja connus en soi comme améliorant les propriétés cosmétiques des cheveux, à savoir notamment ceux décrits dans les demandes de brevet EP-A-337354 et EP-A-557203 et dans les brevets français FR-2 270 846,

2 383 660, 2 598 611, 2 470 596 et 2 519 863.

Les polymères cationiques préférés sont choisis parmi ceux qui contiennent des motifs comportant des groupements amine primaire, secondaire, tertiaire et/ou quaternaire pouvant, soit faire partie de la chaîne principale polymère, soit être portés par un substituant latéral directement relié à celle-ci.

Les polymères cationiques utilisés ont généralement une masse moléculaire moyenne en nombre comprise entre 500 et $5.10^6$ environ, et de préférence comprise entre $10^3$ et $3.10^6$ environ.

Parmi les polymères substantifs cationiques, on peut citer plus particulièrement les polymères du type polyamine, polyaminoamide et polyammonium quaternaire.

Ce sont des produits connus. Ils sont notamment décrits dans les brevets français n° 2 505 348 ou 2 542 997. Parmi lesdits polymères, on peut citer :

(1) les homopolymères ou copolymères dérivés d'esters ou d'amides acryliques ou méthacryliques et comportant au moins un des motifs de formules (II), (III), (IV) ou (V) suivantes:

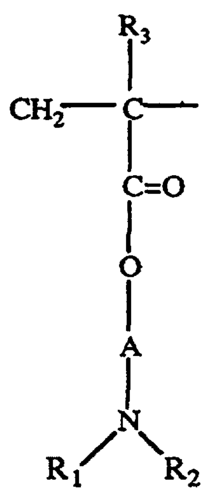
(II)

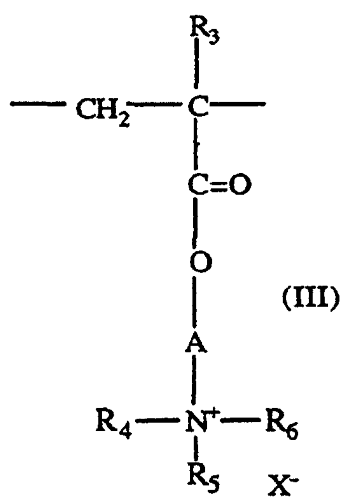
(III)

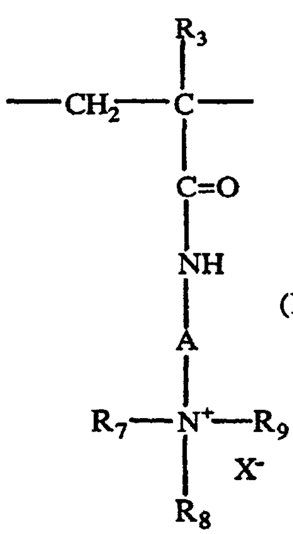
(IV)

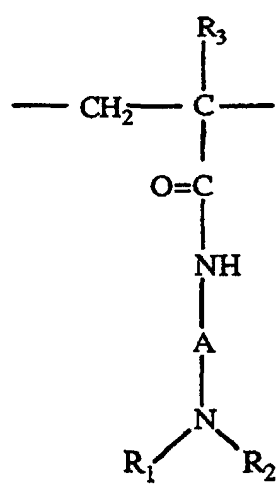
(V)

dans lesquelles:

$R_3$, identiques ou différents, désignent un atome d'hydrogène ou un radical $CH_3$;

A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
$R_4$, $R_5$, $R_6$, identiques ou différents, représentent un groupe alkyle ayant de 1 à 18 atomes de carbone ou un radical benzyle et de préférence un groupe alkyle ayant de 1 à 6 atomes de carbone;
$R_1$ et $R_2$, identiques ou différents, représentent hydrogène ou un groupe alkyle ayant de 1 à 6 atomes de carbone et de préférence méthyle ou éthyle;
X désigne un anion dérivé d'un acide minéral ou organique tel qu'un anion méthosulfate ou un halogénure tel que chlorure ou bromure.

Les polymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs ($C_1$-$C_4$), des acides acryliques ou méthacryliques ou leurs esters, des vinyllactames tels que la vinylpyrrolidone ou le vinylcaprolactame, des esters vinyliques.

Ainsi, parmi ces polymères de la famille (1), on peut citer :

- les copolymères d'acrylamide et de diméthyl-amino-éthyl méthacrylate quaternisé au sulfate de diméthyle ou avec un halogénure de diméthyle,
- les copolymères d'acrylamide et de chlorure de méthacryloyloxyéthyltriméthylammonium décrits par exemple dans la demande de brevet EP-A-080976,
- le copolymère d'acrylamide et de méthosulfate de méthacryloyloxy-éthyltriméthyl-ammonium,
- les copolymères vinylpyrrolidone/acrylate ou méthacrylate de dialkylaminoalkyle quaternisés ou non. Ces polymères sont décrits en détail dans les brevets français 2.077.143 et 2.393.573,
- les terpolymères méthacrylate de diméthylaminoéthyle/vinylcaprolactame/vinylpyrrolidone,
- les copolymère vinylpyrrolidone/méthacrylamidopropyldimethylamine,
- et les copolymères vinylpyrrolidone/méthacrylamide de diméthylaminopropyle quaternisés.

(2) Les dérivés d'éthers de cellulose comportant des groupements ammonium quaternaire décrits dans le brevet français 1 492 597. Ces polymères sont également définis dans le dictionnaire CTFA comme des ammonium quaternaires d'hydroxyéthylcellulose ayant réagi avec un époxyde substitué par un groupement triméthylammonium.

(3) Les dérivés de cellulose cationiques tels que les copolymères de cellulose ou les dérivés de cellulose greffés avec un monomère hydrosoluble d'ammonium quaternaire, et décrits notamment dans le brevet US 4 131 576, tels que les hydroxyalkylcelluloses, comme les hydroxyméthyl-, hydroxyéthyl- ou hydroxypropylcelluloses greffées notamment avec un sel de méthacryloyléthyltriméthylammonium, de méthacrylmidopropyltriméthylammonium, de diméthyldiallylammonium.

(4) Les polysaccharides cationiques décrits plus particulièrement dans les brevets US 3 589 578 et 4 031 307 tel que les gommes de guar contenant des groupements cationiques trialkylammonium. On utilise par exemple des gommes de guar modifiées par un sel (par ex. chlorure) de 2,3-époxypropyl triméthylammonium.

(5) Les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361.

(6) Les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épihalogénhydrine, d'un diépoxyde ou d'un dérivé bis-insaturé ; l'agent réticulant étant utilisé dans des proportions allant de 0,025 à 0,35 mole par groupement amine du polyaminoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508 .

(7) Les dérivés de polyaminoamides résultant de la condensation de polyalcoylènes polyamines avec des acides polycarboxyliques suivie d'une alcoylation par des agents bifonctionnels. On peut citer par exemple les polymères

acide adipique-dialcoylaminohydroxyalcoyldialcoylène triamine dans lesquels le radical alcoyle comporte de 1 à 4 atomes de carbone et désigne de préférence méthyle, éthyle, propyle. De tels polymères sont notamment décrits dans le brevet français 1.583.363.

Parmi ces dérivés, on peut citer plus particulièrement les polymères acide adipique/diméthylaminohydroxypropyl/diéthylène triamine vendus sous la dénomination "Cartaretine F, F4 ou F8" par la société Sandoz.

(8) Les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire du polyalkylène polyamine à l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.

(9) Les cyclopolymères d'alkyl diallyl amine ou de dialkyldiallylammonium tels que les homopolymères ou copolymères comportant comme constituant principal de la chaîne des motifs répondant aux formules (VI) ou (VII) :

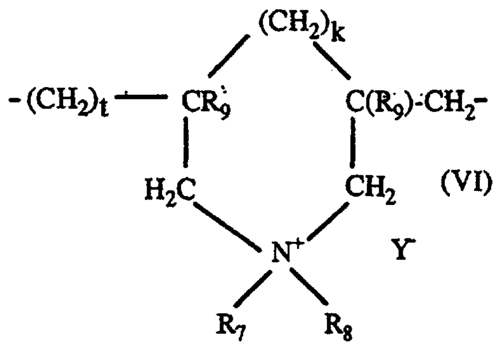
(VI)

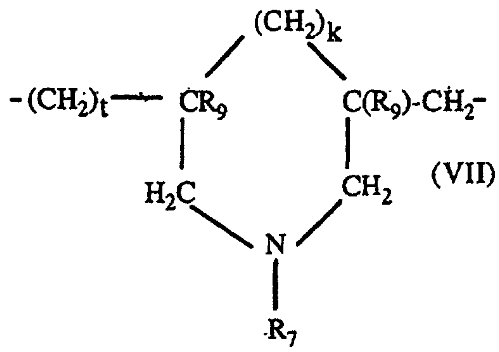
(VII)

formules dans lesquelles k et t sont égaux à 0 ou 1, la somme k + t étant égale à 1 ; $R_9$ désigne un atome d'hydrogène ou un radical méthyle ; $R_7$ et $R_8$, indépendamment l'un de l'autre, désignent un groupement alkyle ayant de 1 à 22 atomes de carbone, un groupement hydroxyalkyle dans lequel le groupement alkyle a de préférence 1 à 5 atomes de carbone, un groupement amidoalkyle inférieur ($C_1$-$C_4$), ou $R_7$ et $R_8$ peuvent désigner conjointement avec l'atome d'azote auquel ils sont rattachés, des groupement hétérocycliques, tels que pipéridinyle ou morpholinyle ; $Y^-$ est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.

(10) Le polymère de diammonium quaternaire contenant des motifs récurrents répondant à la formule :

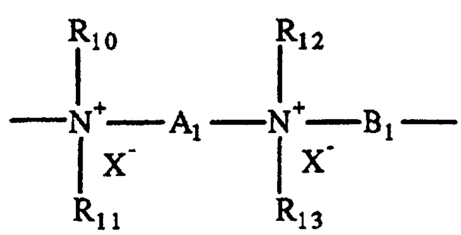
(VIII)

dans laquelle $R_{10}$, $R_{11}$, $R_{12}$ et $R_{13}$, identiques ou différents, représentent des radicaux aliphatiques, alicycliques, ou arylaliphatiques contenant de 1 à 20 atomes de carbone ou des radicaux hydroxyalkylaliphatiques inférieurs, ou bien $R_{10}$, $R_{11}$, $R_{12}$ et $R_{13}$, ensemble ou séparément, constituent avec les atomes d'azote auxquels ils sont rattachés des hétérocycles contenant éventuellement un second hétéroatome autre que l'azote ou bien $R_{10}$, $R_{11}$, $R_{12}$ et $R_{13}$ représentent un radical alkyle en $C_1$-$C_6$ linéaire ou ramifié substitué par un groupement nitrile, ester,

acyle, amide ou -CO-O-$R_{14}$-D ou -CO-NH-$R_{14}$-D où $R_{14}$ est un alkylène et D un groupement ammonium quaternaire ;

$A_1$ et $B_1$ représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
X désigne un anion dérivé d'un acide minéral ou organique;
$A_1$, $R_{10}$ et $R_{12}$ peuvent former avec les deux atomes d'azote auxquels ils sont rattachés un cycle pipérazinique ; en outre si $A_1$ désigne un radical alkylène ou hydroxyalkylène linéaire ou ramifié, saturé ou insaturé, $B_1$ peut également désigner un groupement

$$-(CH_2)_n-CO-D-OC-(CH_2)_n-$$

dans lequel D désigne :

a) un reste de glycol de formule :

-O-Z-O-, où Z désigne un radical hydrocarboné linéaire ou ramifié ou un groupement répondant à l'une des formules suivantes :

$$-(CH_2-CH_2-O)_x-CH_2-CH_2-$$

$$-[CH_2-CH(CH_3)-O]_y-CH_2-CH(CH_3)-$$

où x et y désignent un nombre entier de 1 à 4, représentant un degré de polymérisation défini et unique ou un nombre quelconque de 1 à 4 représentant un degré de polymérisation moyen ;

b) un reste de diamine bis-secondaire tel qu'un dérivé de pipérazine ;
c) un reste de diamine bis-primaire de formule : -NH-Y-NH-, où Y désigne un radical hydrocarboné linéaire ou ramifié, ou bien le radical bivalent

$$-CH_2-CH_2-S-S-CH_2-CH_2- ;$$

d) un groupement uréylène de formule : -NH-CO-NH- ;

De préférence, $X^-$ est un anion tel que le chlorure ou le bromure.
Ces polymères ont une masse moléculaire moyenne en nombre généralement comprise entre 1000 et 100000.

Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.

On peut utiliser plus particulièrement les polymères qui sont constitués de motifs récurrents répondant à la formule (IX) suivante:

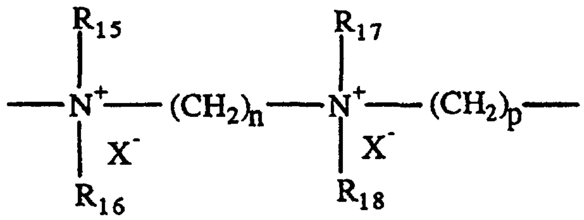

(IX)

dans laquelle $R_{15}$, $R_{16}$, $R_{17}$ et $R_{18}$, identiques ou différents, désignent un radical alkyle ou hydroxyalkyle ayant de 1 à 4 atomes de carbone environ, n et p sont des nombres entiers variant de 2 à 20 environ et, $X^-$ est un anion dérivé d'un acide minéral ou organique.

(11) Les polymères de polyammonium quaternaires constitués de motifs de formule (X):

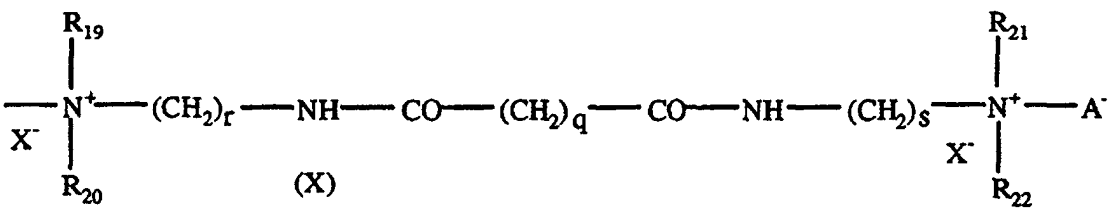

(X)

formule dans laquelle :

$R_{19}$, $R_{20}$, $R_{21}$ et $R_{22}$, identiques ou différents, représentent un atome d'hydrogène ou un radical méthyle, éthyle, propyle, β-hydroxyéthyle, β-hydroxypropyle ou $-CH_2CH_2(OCH_2CH_2)_pOH$, où p est égal à 0 ou à un nombre entier compris entre 1 et 6, sous réserve que $R_{19}$, $R_{20}$, $R_{21}$ et $R_{22}$ ne représentent pas simultanément un atome d'hydrogène,
r et s, identiques ou différents, sont des nombres entiers compris entre 1 et 6,
q est égal à 0 ou à un nombre entier compris entre 1 et 34,
X désigne un atome d'halogène,
A désigne un radical d'un dihalogénure ou représente de préférence $-CH_2-CH_2-O-CH_2-CH_2-$.

De tels composés sont notamment décrits dans la demande de brevet EP-A-122 324.

(12) Les polymères quaternaires de vinylpyrrolidone et de vinylimidazole.

(13) Les polyamines comme le produit référencé sous le nom de POLYETHYLENEGLYCOL (15) TALLOW POLYAMINE dans le dictionnaire CTFA.

(14) Les polymères réticulés de sels de méthacryloyl-oxyalkyl($C_1$-$C_4$) trialkyl($C_1$-$C_4$)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homopolymérisation ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyltriméthylammonium (20/80 en poids) sous forme de dispersion contenant 50 % en poids dudit copolymère dans de l'huile minérale. Cette dispersion est commercialisée sous le nom de " SALCARE® SC 92 " par la Société ALLIED COLLOIDS. On peut également utiliser un homopolymère réticulé du chlorure de méthacryloyloxyéthyl triméthylammonium contenant environ 50 % en poids de l'homopolymère dans de l'huile minérale ou dans un ester liquidé. Ces dispersions sont commercialisées sous les noms de SALCARE® SC 95 et SALCARE® SC 96 par la Société ALLIED COLLOIDS.

D'autres polymères substantifs cationiques utilisables dans le cadre de l'invention sont des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Les polymères substantifs amphotères utilisables conformément à la présente invention peuvent être choisis parmi les polymères comportant des motifs K et M répartis statistiquement dans la chaîne polymère, où K désigne un motif dérivant d'un monomère comportant au moins un atome d'azote basique et M désigne un motif dérivant d'un monomère acide comportant un ou plusieurs groupements carboxyliques ou sulfoniques, ou bien K et M peuvent désigner des groupements dérivant de monomères zwitterioniques de carboxybétaïnes ou de sulfobétaïnes;

K et M peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un radical hydrocarboné, ou bien K et M font partie d'une chaîne d'un polymère à motif éthylène $\alpha,\beta$-dicarboxylique dont l'un des groupements carboxyliques a été amené à réagir avec une polyamine comportant un ou plusieurs groupements amine primaire ou secondaire.

Les polymères filmogènes amphotères répondant à la définition donnée ci-dessus sont choisis notamment parmi les polymères suivants :

(1) Les polymères résultant de la copolymérisation d'un monomère dérivé d'un composé vinylique portant un groupement carboxylique tel que plus particulièrement l'acide acrylique, l'acide méthacrylique, l'acide maléïque, l'acide alpha-chloracrylique, et d'un monomère basique dérivé d'un composé vinylique substitué contenant au moins un atome basique tel que plus particulièrement les méthacrylates et -acrylates de dialkylaminoalkyle, les dialkylaminoalkylméthacrylamide et -acrylamide. De tels composés sont décrits dans le brevet américain n° 3 836 537. On peut également citer le copolymère acrylate de sodium/chlorure d'acrylamidopropyltrimethylammonium.

Le composé vinylique peut être également un sel de dialkyldiallylammonium tel que le chlorure de diéthyldiallylammonium.

(2) Les polymères comportant des motifs dérivant :

a) d'au moins un monomère choisi parmi les acrylamides ou les méthacrylamides substitués sur l'azote par un radical alkyle,
b) d'au moins un comonomère acide contenant un ou plusieurs groupements carboxyliques réactifs, et
c) d'au moins un comonomère basique tel que des esters à substituants amine primaire, secondaire, tertiaire et quaternaire des acides acrylique et méthacrylique et le produit de quaternisation du méthacrylate de diméthylaminoéthyle avec le sulfate de diméthyle ou de diéthyle.

Les acrylamides ou méthacrylamides N-substitués plus particulièrement préférés selon l'invention sont les groupements dont les radicaux alkyle contiennent de 2 à 12 atomes de carbone et plus particulièrement le N-éthylacrylamide, le N-tertiobutylacrylamide, le N-tertiooctylacrylamide, le N-octylacrylamide, le N-décylacrylamide, le N-dodécylacrylamide ainsi que les méthacrylamides correspondants.

Les comonomères acides sont choisis plus particulièrement parmi les acides acrylique, méthacrylique, crotonique, itaconique, maléïque, fumarique ainsi que les monoesters d'alkyle ayant 1 à 4 atomes de carbone des acides ou des anhydrides maléïque ou fumarique.

Les comonomères basiques préférés sont des méthacrylates d'aminoéthyle, de butylaminoéthyle, de N,N'-diméthylaminoéthyle, de N-tertio-butylaminoéthyle.

On utilise particulièrement les copolymères dont la dénomination CTFA ($4^{ème}$ édition, 1991) est Octylacrylamide/acrylates/butylaminoethylmethacrylate copolymer.

(3) Les polyaminoamides réticulés et alcoylés partiellement ou totalement dérivant de polyaminoamides de formule générale :

$$-\!\!\left[ CO - R_{23} - CO - Z \right]\!\!- \qquad (XI)$$

dans laquelle $R_{23}$ représente un radical divalent dérivé d'un acide dicarboxylique saturé, d'un acide aliphatique mono ou dicarboxylique à double liaison éthylénique, d'un ester d'un alcanol inférieur ayant 1 à 6 atome de carbone de ces acides ou d'un radical dérivant de l'addition de l'un quelconque desdits acides avec une amine bis primaire ou bis secondaire, et Z désigne un radical d'une polyalkylène-polyamine bis-primaire, mono ou bis-secondaire et de préférence représente :

a) dans les proportions de 60 à 100 moles %, le radical

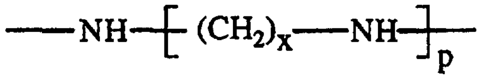

(XII)

où x=2 et p=2 ou 3, ou bien x=3 et p=2,
ce radical dérivant de la diéthylène triamine, de la triéthylène tétraamine ou de la dipropylène triamine;
b) dans les proportions de 0 à 40 moles % le radical (XII) ci-dessus, dans lequel x=2 et p=1 et qui dérive de l'éthylènediamine, ou le radical dérivant de la pipérazine :

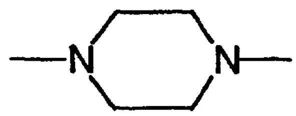

c) dans les proportions de 0 à 20 moles % le radical

$$-NH-(CH_2)_6-NH-$$

dérivant de l'hexaméthylènediamine, ces polyaminoamines étant réticulées par addition d'un agent réticulant bifonctionnel choisi parmi les épihalohydrines, les diépoxydes, les dianhydrides, les dérivés bis insaturés, au moyen de 0,025 à 0,35 mole d'agent réticulant par groupement amine du polyaminoamide et alcoylés par action d'acide acrylique, d'acide chloracétique ou d'une alcane sultone ou de leurs sels.

Les acides carboxyliques saturés sont choisis de préférence parmi les acides ayant 6 à 10 atomes de carbone tels que l'acide adipique, triméthyl-2,2,4-adipique et triméthyl-2,4,4-adipique, téréphtalique, les acides à double liaison éthylénique comme par exemple les acides acrylique, méthacrylique, itaconique.

Les alcanesultones utilisées dans l'alcoylation sont de préférence la propanesultone ou la butanesultone, les sels des agents d'alcoylation sont de préférence les sels de sodium ou de potassium.

(4) Les polymères comportant des motifs zwitterioniques de formule :

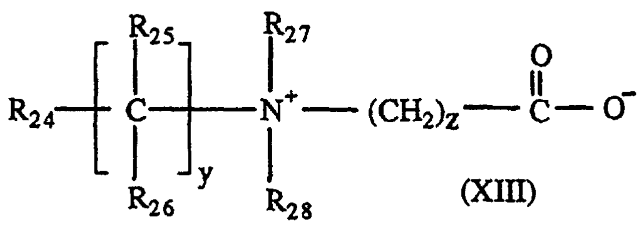

(XIII)

dans laquelle $R_{24}$ désigne un groupement insaturé polymérisable tel qu'un groupement acrylate, méthacrylate, acrylamide ou méthacrylamide, y et z représentent un nombre entier de 1 à 3, $R_{25}$ et $R_{26}$ représentent un atome d'hydrogène, méthyle, éthyle ou propyle, $R_{27}$ et $R_{28}$ représentent un atome d'hydrogène ou un radical alkyle de telle façon que la somme des atomes de carbone dans $R_{27}$ et $R_{28}$ ne dépasse pas 10.

Les polymères comprenant de telles unités peuvent également comporter des motifs dérivés de monomères non zwitterioniques tels que l'acrylate ou le méthacrylate de diméthyl- ou diéthylaminoéthyle ou des acrylates ou méthacrylates d'alkyle, des acrylamides ou méthacrylamides ou l'acétate de vinyle.

A titre d'exemple, on peut citer le copolymère de méthacrylate de méthyle/diméthylcarboxy-méthyl-ammonioéthyl-méthacrylate de méthyle.

(5) les polymères dérivés du chitosane comportant des motifs monomères répondant aux formules (XIV), (XV), (XVI) suivantes :

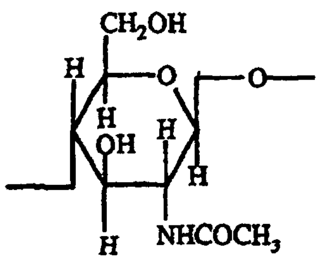

(XIV)

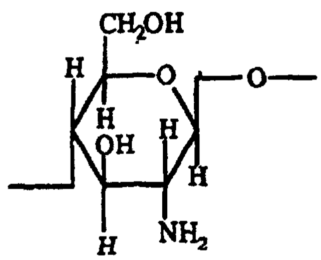

(XV)

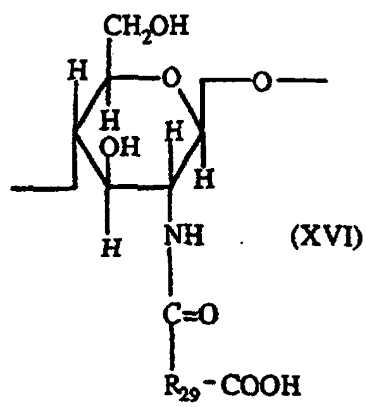

(XVI)

le motif XIV étant présent dans des proportions comprises entre 0 et 30%, le motif XV dans des proportions comprises entre 5 et 50% et le motif XVI dans des proportions comprises entre 30 et 90%, étant entendu que dans ce motif (XVI), $R_{29}$ représente un radical de formule :

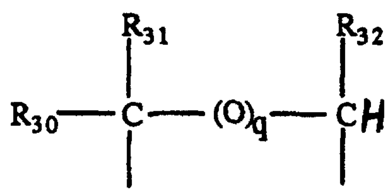

(XVII)

dans laquelle q = 0 ou 1,

si q=0, $R_{30}$, $R_{31}$ et $R_{32}$, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcolylthio, sulfonique, un reste alcoylthio dont le groupe alcoyle porte un reste amino, l'un au moins des radicaux $R_{30}$, $R_{31}$ et $R_{32}$ étant dans ce cas un atome d'hydrogène ;

ou si q=1, $R_{30}$, $R_{31}$ et $R_{32}$ représentent chacun un atome d'hydrogène, ainsi que les sels formés par ces composés avec des bases ou des acides.

(6) Les polymères dérivés de la N-carboxyalkylation du chitosane comme le N-carboxyméthyl chitosane ou le N-carboxybutylchitosane.

(7) Les polymères répondant à la formule générale (XVIII) tels que ceux décrits par exemple dans le brevet français 1 400 366 :

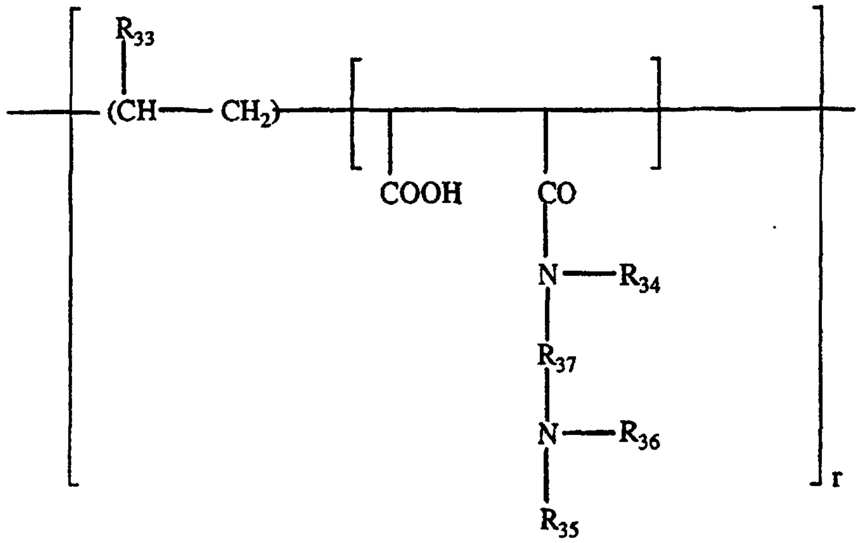

(XVIII)

dans laquelle $R_{33}$ représente un atome d'hydrogène, un radical $CH_3O$, $CH_3CH_2O$, phényle, $R_{34}$ désigne l'hydrogène ou un radical alkyle inférieur tel que méthyle, éthyle, $R_{35}$ désigne l'hydrogène ou un radical alkyle inférieur tel que méthyle, éthyle, $R_{36}$ désigne un radical alkyle inférieur tel que méthyle, éthyle ou un radical répondant à la formule :

$-R_{37}-N(R_{35})_2$, $R_{37}$ représentant un groupement $-CH_2-CH_2-$, $-CH_2-CH_2-CH_2-$, $-CH_2-CH(CH_3)-$, et $R_{35}$ ayant les significations mentionnées ci-dessus, ainsi que les homologues supérieurs de ces radicaux et contenant jusqu'à 6 atomes de carbone.

(8) Des polymères amphotères du type -D-X-D-X- choisis parmi:

a) les polymères obtenus par action de l'acide chloracétique ou le chloracétate de sodium sur les composés comportant au moins un motif de formule :

$$-D-X-D-X-D- \qquad (XIX)$$

où D désigne un radical

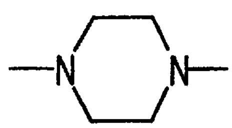

et X désigne le symbole E ou E', E ou E' identiques ou différents désignent un radical bivalent qui est un radical alkylène à chaîne droite ou ramifiée comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques; les atomes d'oxygène, d'azote et de soufre étant présents sous forme de groupements éther, thioéther, sulfoxyde, sulfone, sulfonium, alkylamine, alkénylamine, des groupements hydroxyle, benzylamine, oxyde d'amine, ammonium quaternaire, amide, imide, alcool, ester et/ou uréthanne ;

b) les polymères de formule :

$$-D-X-D-X- \qquad (XX)$$

où D désigne un radical

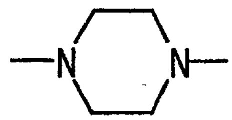

et X désigne le symbole E ou E' et au moins une fois E'; E ayant la signification indiquée ci-dessus et E' est un radical bivalent qui est un radical alkylène à chaîne droite ou ramifiée ayant jusqu'à 7 atomes de carbone dans la chaîne principale, substitué ou non par un ou plusieurs radicaux hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.

(9) Les copolymères alkyl($C_1$-$C_5$)-vinyléther/anhydride maléique modifié partiellement par semiamidification avec une N,N-dialkylaminoalkylamine telle que la N,N-diméthylaminopropylamine ou par semiestérification avec une N,N-dialcanolamine. Ces copolymères peuvent également comporter d'autres comonomères vinyliques tels que le vinylcaprolactame.

Parmi les polymères substantifs cationiques ou amphotères utilisables selon l'invention, on préfère notamment :

- l'homopolymère de chlorure de diméthyldiallylammonium vendu sous la dénomination MERQUAT 100 DRY par la société MERCK;
- les copolymères de chlorure de diméthyldiallylammonium et d'acrylamide vendus sous la dénomination MERQUAT 2200 par la société CALGON;
- les polymères de type poly(ammonium quaternaire) préparés et décrits dans le brevet français 2 270 846, constitués de motifs récurrents répondant à la formule (XXI) suivante :

(XXI) 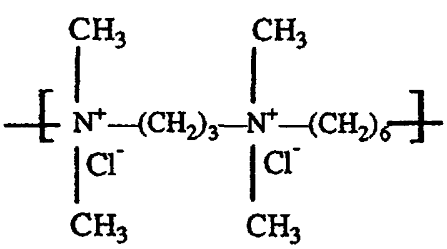

et notamment ceux dont la masse molaire moyenne en poids, déterminée par chromatographie par perméation de gel, est comprise entre 9500 et 9900 ;
- les polymères de type poly(ammonium quaternaire) préparés et décrits dans le brevet français 2 270 846, constitués de motifs récurrents répondant à la formule (XXII) suivante :

(XXII) 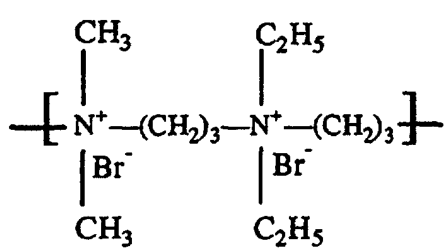

et notamment ceux dont la masse molaire moyenne en poids, déterminée par chromatographie par perméation de gel, est d'environ 1200 ;
- les polymères de type poly(ammonium quaternaire) décrits dans les brevets US 4 390 689, 4 702 906, 4 719 282 et constitués de motifs récurrents répondant à la formule (XXIII) suivante :

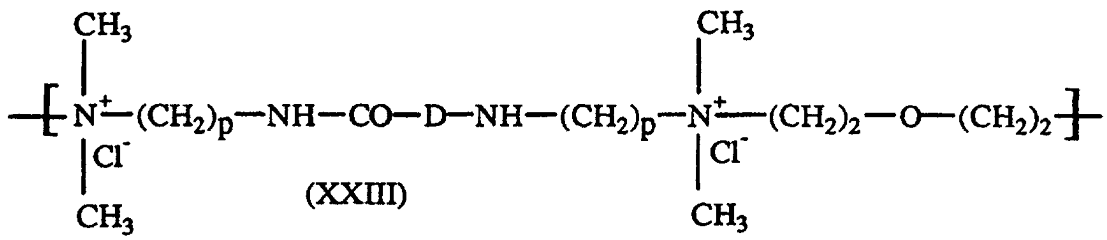

(XXIII)

dans laquelle

p est un nombre entier valant entre 1 et 6,
D représente une simple liaison ou un groupe $-(CH_2)_r-CO-$ où r vaut 4 ou 7,
et notamment ceux dont la masse molaire moyenne en poids est inférieure à 100 000, de préférence inférieure ou égale à 50 000;

- les copolymères amphotères suivants :
- le copolymère chlorure de diallyldiméthylammonium/acide acrylique (80/20) commercialisé sous la dénomination MERQUAT 280 DRY par la société CALGON (dénomination CTFA : Polyquaternium-22);
- le copolymère chlorure de diméthyldiallylammonium/acide acrylique (95/5) vendu sous la dénomination MERQUAT 295 DRY par la société CALGON (dénomination CTFA : Polyquaternium-22);
- le copolymère de chlorure de méthacrylamidopropyltrimonium, d'acide acrylique et d'acrylate de méthyle, commercialisé sou la dénomination MERQUAT 2001 par la société CALGON (dénomination CTFA : Polyquaternium-47) ; et
- le terpolymère acrylamide/chlorure de diméthyldiallylammonium/acide acrylique commercialisé sous la dénomination MERQUAT PLUS 3330 DRY par la société CALGON (dénomination CTFA : Polyquaternium-39) .

Dans la liste des polymères substantifs ci-dessus, on préfère tout particulièrement les copolymères amphotères Polyquaternium-22, Polyquaternium-39 et Polyquaternium-47 (dénominations CTFA).
Selon l'invention, le ou les polymères substantifs cationiques ou amphotères peuvent représenter de 0,03 % à 30 % du poids total de la composition.
Les compositions de l'invention comprennent de préférence au moins un agent tensio-actif.
Les tensio-actifs convenant à la mise en oeuvre de la présente invention sont notamment les suivants :

(i) Tensio-actif(s) anionique(s) :

A titre d'exemple de tensio-actifs anioniques utilisables, seuls ou mélangés, dans le cadre de la présente invention, on peut citer notamment les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, $\alpha$-oléfine-sulfonates, paraffine-sulfonates ; les alkyl($C_6$-$C_{24}$) sulfosuccinates, les alkyl($C_6$-$C_{24}$) éthersulfosuccinates, les alkyl($C_6$-$C_{24}$) amidesulfosuccinates ; les alkyl($C_6$-$C_{24}$) sulfoacétates ; les acyl($C_6$-$C_{24}$) sarcosinates et les acyl($C_6$-$C_{24}$) glutamates. On peut également utiliser les esters d'alkyl($C_6$-$C_{24}$) polyglycosides carboxyliques tels que les alkylglucoside citrates, les alkylpolyglycoside tartrate et les alkylpolyglycoside sulfosuccinates, les alkylsulfosuccinamates ; les acyliséthionates et les N-acyltaurates, le radical alkyle ou acyle de tous ces différents composés comportant de préférence de 12 à 20 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser les acides d'alkyl D galactoside uroniques et leurs sels, les acides alkyl ($C_6$-$C_{24}$) éther carboxyliques polyoxyalkylénés, les acides alkyl($C_6$-$C_{24}$)aryl éther carboxyliques polyoxyalkylénés, les acides alkyl($C_6$-$C_{24}$) amido éther carboxyliques polyoxyalkylénés et leurs sels, en particulier ceux comportant de 2 à 50 groupements oxyde d'alkylène en particulier d'éthylène, et leurs mélanges.

(ii) Tensio-actif(s) non ionique(s) :

Les agents tensio-actifs non-ioniques sont, eux aussi, des composés bien connus en soi (voir notamment à

cet égard "Handbook of Surfactants" par M.R. PORTER, éditions Blackie & Son (Glasgow and London), 1991, pp 116-178) et leur nature ne revet pas, dans le cadre de la présente invention, de caractère critique. Ainsi, ils peuvent être notamment choisis parmi les alcools, les alpha-diols, les alkylphénols ou les acides gras polyéthoxylés, polypropoxylés, ayant une chaîne grasse comportant par exemple 8 à 18 atomes de carbone, le nombre de groupements oxyde d'éthylène ou oxyde de propylène pouvant aller notamment de 2 à 50. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl ($C_{10-14}$) amines ou les oxydes de N-acylaminopropylmorpholine. On notera que les alkylpolyglycosides constituent des tensio-actifs non-ioniques rentrant particulièrement bien dans le cadre de la présente invention.

(iii) Tensio-actif(s) amphotère(s) ou zwitterionique(s) :

Les agents tensio-actifs amphotères ou zwitterioniques, dont la nature ne revêt pas dans le cadre de la présente invention de caractère critique, peuvent être notamment des dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant (par exemple carboxylate, sulfonate, sulfate, phosphate ou phosphonate) ; on peut citer encore les alkyl ($C_8$-$C_{20}$) bétaïnes, les sulfobétaïnes, les alkyl ($C_8$-$C_{20}$) amidoalkyl ($C_1$-$C_6$) betaïnes ou les alkyl ($C_8$-$C_{20}$) amidoalkyl ($C_1$-$C_6$) sulfobétaïnes.

Parmi les dérivés d'amines, on peut citer les produits décrits dans les brevets US-2 528 378 et US-2 781 354 et classés dans le dictionnaire CTFA, 3ème édition, 1982, sous les dénominations Amphocarboxyglycinates et Amphocarboxypropionates de structures respectives :

$$R_{38}\text{-CONHCH}_2\text{CH}_2\text{-N}(R_{39})(R_{40})(\text{CH}_2\text{COO-})$$

dans laquelle : $R_{38}$ désigne un radical alkyle d'un acide $R_{38}$-COOH présent dans l'huile de coprah hydrolysée, un radical heptyle, nonyle ou undécyle, $R_{39}$ désigne un groupement bêta-hydroxyéthyle et $R_{40}$ un groupement carboxyméthyle ;

et

$$R_{38}'\text{-CONHCH}_2\text{CH}_2\text{-N(B)(C)}$$

dans laquelle :

B représente $-CH_2CH_2OX'$,
C représente $-(CH_2)_z$ -Y', avec z = 1 ou 2,
X' désigne le groupement $-CH_2CH_2$-COOH ou un atome d'hydrogène
Y' désigne -COOH ou le radical $-CH_2$-CHOH-$SO_3H$
$R_{38}'$ désigne un radical alkyle d'un acide R-COOH présent dans l'huile de coprah ou dans l'huile de lin hydrolysée, un radical alkyle, notamment en $C_7$, $C_9$, $C_{11}$ ou $C_{13}$, un radical alkyle en $C_{17}$ et sa forme iso, un radical $C_{17}$ insaturé.

Ces composés sont classés dans le dictionnaire CTFA, 5ème édition, 1993, sous les dénominations Disodium Coco ampho-diacetate, Disodium Lauroampho-diacetate, Disodium Capryl ampho-diacetate, Disodium Capryloampho-diacetate, Disodium Coco ampho-dipropionate, Disodium Lauroampho-dipropionate, Disodium Capryl-ampho-dipropionate, Disodium Capryloampho-dipropionate, Lauro-ampho-dipropionic acid, Coco ampho-dipropionic acid.

(iv) Tensio-actifs cationiques :

Parmi les tensio-actifs cationiques on peut citer en particulier : les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyltrialkylammonium, de trialkylbenzylammonium, de trialkylhydroxyalkyl ammonium ou d'alkylpyridinium; les dérivés d'imidazoline ; ou les oxydes d'amines à caractère cationique.

Les quantités d'agents tensioactifs présents dans la composition selon l'invention peuvent représenter de

0,01 à 40% et de préférence de 0,1 à 30% du poids total de la composition.

La composition décolorante anhydre peut se présenter sous forme d'une poudre donnant naissance, après mélange avec de l'eau oxygénée, à un cataplasme. Elle peut également se présenter sous forme d'une crème anhydre décolorante contenant des agents pulvérulents en suspension ou en dispersion dans un support organique, telle que les crèmes décrites dans les brevets US 4 170 637, DE 3 814 356, DE 3 844 956, EP 0 778 020 et DE 1 972 3538 .

Selon la présente invention, la composition décolorante anhydre se présente de préférence sous forme d'une poudre de particules enrobées, non enrobées ou granulées.

La présente invention à également pour objet un procédé de décoloration des fibres kératiniques, en particulier des cheveux humains.

Ce procédé comprend les étapes consistant

- à mélanger, immédiatement avant emploi, la composition décolorante anhydre contenant au moins un agent alcalin, au moins un sel peroxygéné, et l'association d'au moins un polymère épaississant hydrosoluble et d'au moins un polymère amphiphile non ionique comportant au moins une chaîne grasse et éventuellement un polymère amphiphile anionique comportant au moins une chaîne grasse, avec une composition aqueuse de peroxyde d'hydrogène,
- à appliquer le mélange sur la zone des fibres kératiniques à décolorer,
- à laisser reposer pendant un temps suffisant pour obtenir la décoloration recherchée, temps généralement compris entre 10 minutes et une heure, de préférence entre 10 et 45 minutes, et
- à éliminer le mélange de décoloration par rinçage à l'eau, suivi d'un lavage avec un shampooing, puis d'un séchage.

L'invention a en outre pour objet l'utilisation d'une composition décolorante anhydre décrite ci-dessus pour la préparation d'une composition décolorante prête-à-l'emploi. Pour cela la composition anhydre est mélangée avec environ 0,5 à 10 équivalents en poids d'une composition aqueuse de peroxyde d'hydrogène, par exemple une solution, une émulsion ou un gel, ayant une concentration pondérale comprise entre 2 et 12 %. Ce mélange doit se faire immédiatement avant l'application du produit sur les cheveux.

Le pH de la composition décolorante prête à l'emploi est de préférence compris entre 7 et 12 et encore plus préférentiellement entre 8,5 et 11,5.

Un autre objet de l'invention est un dispositif d'emballage en plusieurs parties, également appelé "kit" d'emballage, comprenant au moins deux compartiments dont l'un contient une composition décolorante anhydre telle que décrite ci-dessus, et l'autre une composition aqueuse de peroxyde d'hydrogène.

Les exemples, donnés ci-après à titre purement illustratif et non limitatif, permettront de mieux comprendre l'invention.

**Exemple 1**

On a préparé deux compositions de décoloration pulvérulentes dont l'une (composition **A**) contenait une combinaison de deux épaississants hydrosolubles classiques, à savoir l'hydroxyéthylcellulose et un polymère naturel, la gomme de guar, et l'autre (composition **B** selon l'invention) contenait l'association d'un épaississant hydrosoluble classique, c'est-à-dire l'hydroxyéthylcellulose, et d'un polymère amphiphile non ionique selon l'invention, à savoir la cétylhydroxyéthylcellulose.

Le tableau ci-dessous présente la nature et les quantités pondérales des ingrédients de ces deux compositions.

| | quantités (en % en poids) | |
|---|---|---|
| | **composition A** (art antérieur) | **composition B** (selon l'invention) |
| persulfate de potassium | 35 | 35 |
| persulfate de sodium | 30 | 30 |
| métasilicate de sodium | 14 | 14 |
| chlorure d'ammonium | 5 | 5 |
| EDTA | 1 | 1 |
| dioctylsulfosuccinate de Na/ benzoate de sodium | 1 | 1 |
| stéarate de calcium | 1 | 1 |
| silice | 7 | 7 |
| gomme de guar * | 3 | 0 |
| cétylhydroxyéthylcellulose** | 0 | 3 |
| hydroxyéthylcellulose*** | 3 | 3 |

* vendue sous la dénomination GUARGEL D/15 par la Société Française des Colloïdes

** vendue sous la dénomination POLYSURF 67 par la société HERCULES

*** vendue sous la dénomination CELLOSIZE POLYMER PCG-10 par la société AMERCHOL

On a mélangé 40 g de chacune des compositions **A** et **B** avec 80 g de la composition **C** suivante de manière à obtenir deux compositions de décoloration prêtes-à-l'emploi **AC** et **BC**.

| | Composition C (quantités en % en poids) |
|---|---|
| alcool cétéarylique/ceteareth-30 | 2,85 |
| stabilisants | 0,06 |
| séquestrant | 0,15 |
| peroxyde d'hydrogène | 9 |
| acide phosphorique | qsp pH 2 |
| eau distillée | qsp 100 |

On a procédé à des mesures viscosimétriques au cours du temps à l'aide d'un viscosimètre rotatif (modèle RHEOMAT RM 180, société METTLER). Toutes les mesures ont été effectuées à 25 °C, à l'aide du mobile n° 3, après 30 secondes de cisaillement à une vitesse de 50 $s^{-1}$.

Avant chaque mesure, la composition AC ou BC a été homogénéisée par une agitation faible d'une durée de 1 minute.

La perte de viscosité a été calculée selon la formule suivante :

$$\text{perte (\%)} = ((\eta_5 - \eta_t) / \eta_5) \times 100$$

où

$\eta_5$ est la viscosité mesurée 5 minutes après mélange des compositions A et B avec la composition C, et
$\eta_t$ est la viscosité mesurée au bout d'un temps **t** (en minutes) suivant le mélange des compositions A et B avec la composition C.

Les résultats obtenus ont été les suivants :

| t (min) | composition **AC** (art antérieur) | | composition **BC** (invention) | |
|---|---|---|---|---|
| | viscosité (Pa.s) | perte (%) | viscosité (Pa.s) | perte (%) |
| 5 | 11,54 | | 15,85 | |
| 10 | 6,64 | **42** | 10,45 | **34** |
| 20 | 3,99 | **65** | 9,95 | **37** |
| 30 | 3,32 | **71** | 10,32 | **35** |

Pour la composition AC correspondant à l'état de la technique, on a constaté une perte de viscosité croissante au cours du temps qui a atteint 70 % au bout de seulement 25 minutes.

Pour la composition BC selon la présente invention, la viscosité diminue d'environ 35 % au bout de 10 minutes mais reste ensuite constante au cours du temps.

**Exemple 2**

On a préparé la composition de décoloration sous forme de crème anhydre suivante (quantités en % en poids) :

| | |
|---|---|
| Palmitate d'isopropyle | 23 |
| Huile minérale | 3 |
| Persulfate de potassium. | 25 |
| Persulfate de sodium | 20 |
| Métasilicate de sodium | 12 |
| Alginate de sodium | 2 |
| Chlorure d'ammonium | 4 |
| Acide éthylène diamine tétraacétique | 1 |
| Alcool cétylstéarylique à 25 moles d'oxyde d'éthylène | 2 |
| Argile | 1 |
| Polyquaternium-22 | 1 |
| Serad FX-1100 | 3 |
| Oxyde de titane | 1 |
| Stéarate de magnésium | 2 |

10g de cette composition ont été mélangés avec 15g de la composition C décrite précédemment. On a appliqué et maintenu ce mélange sur des cheveux à décolorer pendant 45 minutes. On a obtenu une décoloration uniforme après rinçage, shampooing et séchage. L'état de la fibre était satisfaisant avec une dégradation limitée.

## Revendications

**1.** Composition anhydre pour la décoloration de fibres kératiniques, en particulier de fibres kératiniques humaines, comprenant dans un milieu approprié pour la décoloration, au moins un agent alcalin et de 20 à 70% en poids au moins un sel peroxygéné, **caractérisée par le fait qu'**elle contient en outre l'association

- d'au moins un polymère épaississant hydrosoluble et
- de 0,03 à 30% en poids d'au moins un polymère amphiphile non ionique comportant au moins une chaîne grasse.

2. Composition selon la revendication 1, **caractérisée par le fait que** le polymère épaississant hydrosoluble est un polymère d'origine naturelle ou synthétique.

3. Composition selon la revendication 2, **caractérisée par le fait que** ledit polymère épaississant synthétique est choisi parmi la polyvinylpyrrolidone, l'acide polyacrylique, le polyacrylamide, l'acide polyacrylamidométhylpropanesulfonique ou des copolymères de ceux-ci, réticulés ou non réticulés.

4. Composition selon la revendication 2, **caractérisée par le fait que** ledit polymère épaississant d'origine naturelle est choisi parmi

   (a) les gommes de guar non-ioniques ;
   (b) les gommes de biopolysaccharides d'origine microbienne telles que les gommes de scléroglucane et de xanthane ;
   (c) les gommes issues d'exsudats végétaux telles que la gomme arabique, la gomme ghatti, la gomme karaya ou la gomme adragante ;
   (d) les gommes extraites d'algues telles que les carraghénanes ou l'agar,
   (e) les gommes issues d'extraits végétaux tels que la gomme de caroube ou les pectines extraites de pulpes de fruits ;
   (f) les alginates ;
   (g) les amidons ; et
   (h) les hydroxyalkylcelluloses et carboxyalkylcelluloses.

5. Composition selon la revendication 4, **caractérisée par le fait que** ledit polymère épaississant d'origine naturelle est une gomme de guar, un dérivé de gomme de guar ou une hydroxyalkylcellulose.

6. Composition selon l'une quelconque des revendications précédentes, **caractérisée par le fait que** ledit polymère amphiphile non ionique comportant au moins une chaîne grasse est choisi parmi

   - les celluloses ou hydroxyalkylcelluloses modifiées par des groupements comportant au moins une chaîne grasse de type alkyle, arylalkyle ou alkylaryle contenant un groupe alkyle en $C_{8-22}$, ou par des groupes alkylphénol polyalcoxylés ;
   - les hydroxypropylguars modifiés par des groupements comportant au moins une chaîne grasse en $C_{8-22}$ ;
   - les polyuréthannes comportant au moins une chaîne grasse de type alkyle ou alcényle en $C_{8-30}$ ;
   - les copolymères de vinylpyrrolidone et de monomères hydrophobes à chaîne grasse,
   - les copolymères de (méth)acrylates d'alkyle en $C_{1-6}$ et de monomères amphiphiles comportant au moins une chaîne grasse,
   - les copolymères de (méth)acrylates hydrophiles et de monomères hydrophobes comportant au moins une chaîne grasse.

7. Composition selon la revendication 6, **caractérisée par le fait que** le polymère amphiphile non ionique est une hydroxyéthylcellulose modifiée par des groupements comportant au moins un radical alkyle en $C_{8-22}$ ou un polyuréthanne comportant au moins une chaîne alkyle en $C_{10-20}$.

8. Composition selon l'une quelconque des revendications précédentes, **caractérisée par le fait que** le polymère épaississant hydrosoluble est présent à raison de 0,03 à 30 % en poids, de préférence à raison de 0,3 à 15 % en poids, par rapport à la composition anhydre.

9. Composition selon l'une quelconque des revendications précédentes, **caractérisée par le fait que** le polymère amphiphile non ionique comportant au moins une chaîne grasse est présent à raison de 0,3 à 15 % en poids, par rapport à la composition anhydre.

10. Composition selon l'une quelconque des revendications précédentes, **caractérisée par le fait que** le rapport en poids du polymère amphiphile non ionique comportant au moins une chaîne grasse au polymère épaississant hydrosoluble est compris entre 10/1 et 1/10, de préférence entre 5/1 et 1/5.

11. Composition selon l'une quelconque des revendications précédentes, **caractérisée par le fait qu'**elle contient en outre un polymère amphiphile anionique comportant au moins une chaîne grasse.

**12.** Composition selon la revendication 11, **caractérisée par le fait que** le polymère amphiphile anionique comportant au moins une chaîne grasse est un copolymère comprenant

- des motifs hydrophiles dérivés d'un ou de plusieurs monomères à insaturation éthylénique portant une fonction d'acide carboxylique, et
- des motifs hydrophobes dérivés d'un ou de plusieurs monomères à insaturation éthylénique portant une chaîne latérale hydrophobe.

**13.** Composition selon la revendication 11 ou 12, **caractérisée par le fait que** le ou les monomères à insaturation éthylénique portant une fonction d'acide carboxylique sont choisis parmi l'acide éthacrylique, l'acide méthacrylique et l'acide acrylique, de préférence parmi l'acide méthacrylique, l'acide acrylique et des mélanges de ceux-ci.

**14.** Composition selon l'une quelconque des revendications 11 à 13, **caractérisée par le fait que** le ou les monomères à insaturation éthylénique portant une chaîne latérale hydrophobe sont choisis parmi les éthacrylates, méthacrylates et/ou acrylates d'alkyle en $C_{10-30}$, de préférence en $C_{12-22}$.

**15.** Composition selon l'une quelconque des revendications 11 à 13, **caractérisée par le fait que** le ou les monomères à insaturation éthylénique portant une chaîne latérale hydrophobe sont choisis parmi les éthers allyliques d'alcools gras correspondant à la formule

$$(I) \qquad CH_2{=}CR'CH_2{-}O{-}B_n{-}R$$

dans laquelle

R' représente un atome d'hydrogène ou un groupe méthyle,
B représente un groupe éthylèneoxy,
n est un nombre entier valant entre 0 et 100,
R représente un groupe hydrocarboné choisi parmi les restes alkyle, arylalkyle, aryle, alkylaryle ou cycloalkyle comportant de 8 à 30 atomes de carbone, de préférence de 10 à 24 atomes de carbone.

**16.** Composition selon l'une quelconque des revendications 11 à 15, **caractérisée par le fait que** le polymère amphiphile anionique comportant au moins une chaîne grasse comprend en outre des motifs dérivés d'un monomère réticulant contenant deux doubles liaisons éthyléniques non conjuguées.

**17.** Composition anhydre selon la revendication 16, **caractérisé par le fait que** ledit monomère réticulant est choisi parmi le phtalate de diallyle, le (méth)acrylate d'allyle, le divinylbenzène, le diméthacrylate de (poly)éthylèneglycol, le méthylène-bis-acrylamide, le polyallylsucrose ou le polyallylpentaérythritol.

**18.** Composition selon l'une quelconque des revendications 11 à 17, **caractérisée par le fait que** le polymère amphiphile anionique comportant au moins une chaîne grasse est présent à raison de 0,03 à 30 % en poids par rapport à la composition anhydre.

**19.** Composition selon l'une quelconque des revendications précédentes, **caractérisée par le fait que** ledit agent alcalin est choisi parmi les silicates, phosphates ou carbonates de métaux alcalins ou alcalino-terreux, en particulier les métasilicates de métaux alcalins.

**20.** Composition selon l'une quelconque des revendications précédentes, **caractérisée par le fait que** ledit sel peroxygéné est choisi parmi les persulfates, percarbonates et perborates d'ammonium et de métaux alcalins, en particulier parmi les persulfates de sodium et de potassium.

**21.** Composition selon la revendication 20, **caractérisée par le fait qu'**elle contient entre 30 et 60 % en poids, d'au moins un sel peroxygéné.

**22.** Composition selon l'une quelconque des revendications précédentes, **caractérisée par le fait qu'**elle contient en outre des adjuvants de décoloration choisis parmi les agents de contrôle du dégagement d'oxygène, les agents tensioactifs anioniques, non ioniques, cationiques, amphotères ou zwitterioniques et leurs mélanges, les huiles minérales et végétales, les cires, les adjuvants de granulation, les liants, les charges minérales, les opacifiants,

les colorants, les séquestrants, les parfums et les polymères substantifs cationiques et amphotères.

**23.** Composition selon l'une quelconque des revendications précédentes, **caractérisée par le fait qu'**elle contient de 0,01 à 40 % en poids, et de préférence de 0,1 à 30 % en poids d'au moins un agent tensioactif.

**24.** Composition selon l'une quelconque des revendications précédentes, **caractérisée par le fait qu'**elle contient de 0,03 à 30 % en poids d'au moins un polymère substantif cationique ou amphotère.

**25.** Composition selon l'une quelconque des revendications précédentes, **caractérisée par le fait qu'**elle se présente sous forme d'une poudre, ou d'une suspension ou dispersion de poudre dans un support liquide organique anhydre.

**26.** Utilisation de la composition anhydre décolorante selon l'une quelconque des revendications précédentes pour la préparation d'une composition décolorante prête-à-l'emploi par addition d'une composition aqueuse de peroxyde d'hydrogène.

**27.** Procédé de décoloration de fibres kératiniques, en particulier de cheveux humains, comprenant les étapes consistant

- à mélanger, immédiatement avant emploi, une composition décolorante anhydre définie selon l'une quelconque des revendications 1 à 25, avec une composition aqueuse de peroxyde d'hydrogène,
- à appliquer le mélange obtenu sur la zone des fibres kératiniques à décolorer,
- à laisser reposer pendant un temps suffisant pour obtenir la décoloration recherchée, et
- à éliminer le mélange décolorant par rinçage à l'eau suivi d'un lavage avec un shampooing, puis d'un séchage.

**28.** Dispositif à plusieurs compartiments, ou "kit", pour la décoloration des fibres kératiniques et en particulier des fibres kératiniques humaines telles que les cheveux, **caractérisé par le fait qu'**il comporte au moins deux compartiments dont l'un contient une composition anhydre selon l'une quelconque des revendications 1 à 25, et l'autre une composition aqueuse de peroxyde d'hydrogène.

## Claims

**1.** Anhydrous composition for bleaching keratin fibres, in particular human keratin fibres, comprising, in a medium which is suitable for bleaching, at least one alkaline agent and from 20 to 70% by weight of at least one peroxygenated salt, **characterized in that** it contains in addition, a combination

- of at least one water-soluble thickening polymer, and
- from 0.03 to 30% by weight of at least one nonionic amphiphilic polymer comprising at least one fatty chain.

**2.** Composition according to Claim 1, **characterized in that** the water-soluble thickening polymer is a polymer of natural or synthetic origin.

**3.** Composition according to Claim 2, **characterized in that** the said synthetic thickening polymer is chosen from polyvinylpyrrolidone, polyacrylic acid, polyacrylamide, polyacrylamidomethylpropanesulphonic acid or copolymers thereof, these polymers being crosslinked or non-crosslinked.

**4.** Composition according to Claim 2, **characterized in that** the said thickening polymer of natural origin is chosen from

(a) nonionic guar gums;
(b) biopolysaccharide gums of microbial origin such as scleroglucan gum and xanthan gum;
(c) gums derived from plant exudates such as gum arabic, ghatti gum, karaya gum or gum tragacanth;
(d) gums extracted from algae, such as carrageenans or agar,
(e) gums obtained from plant extracts, such as carob gum or pectins extracted from fruit pulp;
(f) alginates;
(g) starches; and
(h) hydroxyalkylcelluloses and carboxyalkylcelluloses.

**5.** Composition according to Claim 4, **characterized in that** the said thickening polymer of natural origin is a guar gum, a guar gum derivative or a hydroxyalkylcellulose.

**6.** Composition according to any one of the preceding claims, **characterized in that** the said nonionic amphiphilic polymer comprising at least one fatty chain is chosen from

- celluloses or hydroxyalkylcelluloses modified with groups comprising at least one fatty chain of alkyl, arylalkyl or alkylaryl type containing a $C_8$-$C_{22}$ alkyl group, or with polyalkoxylated alkylphenol groups;
- hydroxypropyl guars modified with groups comprising at least one $C_8$-$C_{22}$ fatty chain;
- polyurethanes comprising at least one fatty chain of $C_8$-$C_{30}$ alkyl or alkenyl type;
- copolymers of vinylpyrrolidone and of hydrophobic monomers containing a fatty chain;
- copolymers of $C_1$-$C_6$ alkyl (meth)acrylates and of amphiphilic monomers comprising at least one fatty chain,
- copolymers of hydrophilic (meth)acrylates and of hydrophobic monomers comprising at least one fatty. chain.

**7.** Composition according to Claim 6, **characterized in that** the nonionic amphiphilic polymer is a hydroxyethylcellulose modified with groups comprising at least one $C_8$-$C_{22}$ alkyl radical or a polyurethane comprising at least one $C_{10}$-$C_{20}$ alkyl chain.

**8.** Composition according to any one of the preceding claims, **characterized in that** the water-soluble thickening polymer is present in a proportion of from 0.03 to 30% by weight, preferably in a proportion of from 0.3 to 15% by weight, relative to the anhydrous composition.

**9.** Composition according to any one of the preceding claims, **characterized in that** the nonionic amphiphilic polymer comprising at least one fatty chain is present in a proportion of from 0.3 to 15% by weight, relative to the anhydrous composition.

**10.** Composition according to any one of the preceding claims, **characterized in that** the weight ratio of the nonionic amphiphilic polymer comprising at least one fatty chain to the water-soluble thickening polymer is between 10/1 and 1/10, preferably between 5/1 and 1/5.

**11.** Composition according to any one of the preceding claims, **characterized in that** it also contains an anionic amphiphilic polymer comprising at least one fatty chain.

**12.** Composition according to Claim 11, **characterized in that** the anionic amphiphilic polymer comprising at least one fatty chain is a copolymer comprising

- hydrophilic units derived from one or more monomers containing ethylenic unsaturation bearing a carboxylic acid function, and
- hydrophobic units derived from one or more monomers containing ethylenic unsaturation bearing a hydrophobic side chain.

**13.** Composition according to Claim 11 or 12, **characterized in that** the monomer(s) containing ethylenic unsaturation bearing a carboxylic acid function is(are) chosen from ethacrylic acid, methacrylic acid and acrylic acid, preferably from methacrylic acid and acrylic acid and mixtures thereof.

**14.** Composition according to any one of Claims 11 to 13, **characterized in that** the monomer(s) containing ethylenic unsaturation bearing a hydrophobic side chain is (are) chosen from $C_{10}$-$C_{30}$, preferably $C_{12}$-$C_{22}$, alkyl ethacrylates, methacrylates and/or acrylates.

**15.** Composition according to any one of Claims 11 to 13, **characterized in that** the monomer(s) containing ethylenic unsaturation bearing a hydrophobic side. chain is(are) chosen from allyl fatty alkyl ethers corresponding to the formula

$$\text{(I)} \qquad CH_2{=}CR'CH_2\text{-}O\text{-}B_n\text{-}R$$

in which

R' represents a hydrogen atom or a methyl group,
B represents an ethylenoxy group,
n is an integer between 0 and 100,
R represents a hydrocarbon-based group chosen from alkyl, arylalkyl, aryl, alkylaryl and cycloalkyl residues comprising from 8 to 30 carbon atoms, preferably from 10 to 24 carbon atoms.

**16.** Composition according to any one of Claims 11 to 15, **characterized in that** the anionic amphiphilic polymer comprising at least one fatty chain also comprises units derived from a crosslinking monomer containing two non-conjugated ethylenic double bonds.

**17.** Anhydrous composition according to Claim 16, **characterized in that** the said crosslinking monomer is chosen from diallyl phthalate, allyl (meth)acrylate, divinylbenzene, (poly)ethylene glycol dimethacrylate, methylenebisacrylamide, polyallylsucrose or polyallylpentaerythritol.

**18.** Composition according to any one of Claims 11 to 17, **characterized in that** the anionic amphiphilic polymer comprising at least one fatty chain is present in a proportion of from 0.03 to 30% by weight relative to the anhydrous composition.

**19.** Composition according to any one of the preceding claims, **characterized in that** the said alkaline agent is chosen from alkali metal or alkaline-earth metal silicates, phosphates or carbonates, in particular alkali metal metasilicates.

**20.** Composition according to any one of the preceding claims, **characterized in that** the said peroxygenated salt is chosen from the ammonium and alkali metal persulphates, percarbonates and perborates, in particular from sodium persulphate and potassium persulphate.

**21.** Composition according to Claim 20, **characterized in that** it contains between 30 and 60% by weight of at least one peroxygenated salt.

**22.** Composition according to any one of the preceding claims, **characterized in that** it also contains bleaching adjuvants chosen from agents for controlling the release of oxygen, anionic, nonionic, cationic, amphoteric or zwitterionic surfactants and mixtures thereof, mineral or plant oils, waxes, granulating adjuvants, binders, mineral fillers, opacifiers, dyes, sequestering agents, fragrances and cationic or amphoteric substantive polymers.

**23.** Composition according to any one of the preceding claims, **characterized in that** it contains from 0.01 to 40% by weight, and preferably from 0.1 to 30% by weight, of at least one surfactant.

**24.** Composition according to any one of the preceding claims, **characterized in that** it contains from 0.03 to 30% by weight of at least one cationic or amphoteric substantive polymer.

**25.** Composition according to any one of the preceding claims, **characterized in that** it is in the form of a powder, or of a suspension or dispersion of powder in an anhydrous organic liquid support.

**26.** Use of the anhydrous bleaching composition according to any one of the preceding claims to prepare a ready-to-use bleaching composition by addition of an aqueous hydrogen peroxide composition.

**27.** Process for bleaching keratin fibres, in particular human hair, comprising the steps consisting.

- in mixing, immediately before use, an anhydrous bleaching composition defined according to any one of Claims 1 to 25 with an aqueous hydrogen peroxide composition,
- in applying the mixture obtained to the region of keratin fibres to be bleached,
- in leaving the mixture to stand on the fibres for a period which is sufficient to obtain the desired bleaching effect, and
- in removing the bleaching mixture by rinsing with water, followed by washing with a a shampoo, then drying.

**28.** Multi-compartment device or "kit" for bleaching keratin fibres, and in particular human keratin fibres such as the hair, **characterized in that** it comprises at least two compartments one of which contains an anhydrous composition according to any one of Claims 1 to 25, and the other of which contains an aqueous hydrogen peroxide composition.

## Patentansprüche

1. Wasserfreie Zusammensetzung zum Bleichen von Keratinfasern und insbesondere menschlichen Keratinfasern, die in einem zum Bleichen geeigneten Medium mindestens eine Base und 20 bis 70 Gew.-% mindestens ein Peroxidsalz enthält, **dadurch gekennzeichnet, daß** sie ferner die folgende Kombination enthält:

   - mindestens ein wasserlösliches verdickendes Polymer, und
   - 0,03 bis 30 Gew.-% mindestens eines anionischen amphiphilen Polymers, das mindestens eine Fettkette aufweist.

2. Zusammensetzung nach Anspruch 1, **dadurch gekennzeichnet, daß** das wasserlösliche verdickende Polymer ein Polymer natürlicher oder synthetischer Herkunft ist.

3. Zusammensetzung nach Anspruch 2, **dadurch gekennzeichnet, daß** das synthetische verdickende Polymer unter Polyvinylpyrrolidon, Polyacrylsäure, Polyacrylamid, Polyacrylamidomethylpropansulfonsäure oder ihren Copolymeren ausgewählt ist, die gegebenenfalls vernetzt sind.

4. Zusammensetzung nach Anspruch 2, **dadurch gekennzeichnet, daß** das verdickende Polymer natürlicher Herkunft ausgewählt ist unter:

   a. nichtionischen Guargummen;
   b. Gummen von Biopolysacchariden mikrobieller Herkunft, beispielsweise Skleroglucanen und Xanthangummi;
   c. Gummen aus pflanzlichen Exsudaten, wie Gummi arabicum, Ghatti Gummi, Karaya-Gummi und Tragant;
   d. Gummen, die aus Algen extrahiert wurden, wie Carrageenane oder Agar;
   e. Gummen aus Pflanzenextrakten, wie Johannisbrot-Kernmehl, oder Pektinen aus Fruchtfleisch:
   f. Alginaten;
   g. Stärkeverbindungen; und
   h. Hydroxyalkylcellulosen und Carboxyalkylcellulosen.

5. Zusammensetzung nach Anspruch 4, **dadurch gekennzeichnet, daß** es sich bei dem verdickenden Polymer natürlicher Herkunft um Guargummi, ein Guargummiderivat oder Hydroxyalkylcellulose handelt.

6. Zusammensetzung nach einem der vorhergehenden Ansprüche, **dadurch gekennzeichnet, daß** das nichtionische amphiphile Polymer, das mindestens eine Fettkette aufweist, ausgewählt ist unter:

   - Cellulosen oder Hydroxyalkylcellulosen, die mit Gruppen, die mindestens eine Fettkette vom Typ einer Alkyl-, Arylalkyl- oder Alkylarylgruppe aufweisen, die eine $C_{8-22}$-Alkylgruppe enthalten, oder mit polyalkoxylierten Alkylphenolgruppen modifiziert sind;
   - Hydroxypropylguarverbindungen, die mit Gruppen modifiziert sind, die mindestens eine $C_{8-22}$-Fettkette aufweisen;
   - Polyurethanen, die mindestens eine Fettkette vom Alkyltyp oder Alkenyltyp mit 8 bis 30 Kohlenstoffatomen aufweisen;
   - Copolymeren von Vinylpyrrolidon und hydrophoben Monomeren mit Fettkette;
   - Copolymeren von $C_{1-6}$-Alkyl(meth)acrylaten und amphiphilen Monomeren, die mindestens eine Fettkette aufweisen; und
   - Copolymeren von hydrophilen (Meth)acrylaten und hydrophoben Monomeren, die mindestens eine Fettkette aufweisen.

7. Zusammensetzung nach Anspruch 6, **dadurch gekennzeichnet, daß** das nichtionische amphiphile Polymer eine Hydroxyethylcellulose, die mit Gruppen modifiziert ist, die mindestens eine $C_{8-22}$-Alkylgruppe enthalten, oder ein Polyurethan ist, das mindestens eine $C_{10-20}$-Alkylgruppe aufweist.

8. Zusammensetzung nach einem der vorhergehenden Ansprüche, **dadurch gekennzeichnet, daß** das wasserlösliche verdickende Polymer in einer Menge von 0,03 bis 30 Gew.-% und vorzugsweise 0,3 bis 15 Gew.-%, bezogen auf die wasserfreie Zusammensetzung, vorliegt.

9. Zusammensetzung nach einem der vorhergehenden Ansprüche, **dadurch gekennzeichnet, daß** das nichtionische amphiphile Polymer, das mindestens eine Fettkette aufweist, in einer Menge von 0,3 bis 15 Gew.-% vorliegt.

**10.** Zusammensetzung nach einem der vorhergehenden Ansprüche, **dadurch gekennzeichnet, daß** das Gewichtsverhältnis des nichtionischen amphiphilen Polymers, das mindestens eine Fettkette aufweist, und des wasserlöslichen verdickenden Polymers im Bereich von 10/1 bis 1/10 und vorzugsweise 5/1 bis 1/5 liegt.

**11.** Zusammensetzung nach einem der vorhergehenden Ansprüche, **dadurch gekennzeichnet, daß** sie ferner ein anionisches amphiphiles Polymer, das mindestens eine Fettkette aufweist, enthält.

**12.** Zusammensetzung nach Anspruch 11, **dadurch gekennzeichnet, daß** das anionische amphiphile Polymer, das mindestens eine Fettkette aufweist, ein Copolymer ist, das enthält:

- hydrophile Einheiten, die von einem oder mehreren Monomeren mit ethylenischer Doppelbindung abgeleitet sind, die eine Carbonsäuregruppe aufweisen, und
- hydrophobe Einheiten, die von einem oder mehreren Monomeren mit ethylenischer Doppelbindung abgeleitet sind, die eine hydrophobe Seitenkette aufweisen.

**13.** Zusammensetzung nach Anspruch 11 oder 12, **dadurch gekennzeichnet, daß** das Monomer oder die Monomere mit ethylenischer Doppelbindung, die eine Carboxygruppe tragen, unter Ethacrylsäure, Methacrylsäure und Acrylsäure und vorzugsweise Methacrylsäure und Acrylsäure und ihren Gemischen ausgewählt sind.

**14.** Zusammensetzung nach einem der Ansprüche 11 bis 13, **dadurch gekennzeichnet, daß** das Monomer oder die Monomere mit ethylenischer Doppelbindung, die eine hydrophobe Seitenkette aufweisen, unter den Ethacrylaten, Methacrylaten und/oder Acrylaten von $C_{10-30}$-Alkyl und vorzugsweise $C_{12-22}$-Alkyl ausgewählt sind.

**15.** Zusammensetzung nach einem der Ansprüche 11 bis 13, **dadurch gekennzeichnet, daß** das Monomer oder die Monomere mit ethylenischer Doppelbindung, die eine hydrophobe Seitenkette aufweisen, unter den Allylethern von Fettalkoholen der folgenden Formel ausgewählt sind:

$$\text{(I)} \qquad CH_2{=}CR'CH_2\text{-}O\text{-}B_n\text{-}R$$

worin bedeuten:

R' ein Wasserstoffatom oder die Methylgruppe,
B Ethylenoxy,
n Null oder eine ganze Zahl von 1 bis 100,
R eine Kohlenwasserstoffgruppe, die unter Alkyl, Arylalkyl, Aryl, Alkylaryl oder Cycloalkyl mit 8 bis 30 Kohlenstoffatomen und vorzugsweise 10 bis 24 Kohlenstoffatomen ausgewählt ist.

**16.** Zusammensetzung nach einem der Ansprüche 11 bis 15, **dadurch gekennzeichnet, daß** das anionische amphiphile Polymer, das mindestens eine Fettkette aufweist, ferner Einheiten enthält, die von einem vernetzenden Monomer abgeleitet sind, das nicht konjugierte ethylenische Doppelbindungen enthält.

**17.** Zusammensetzung nach Anspruch 16, **dadurch gekennzeichnet, daß** das vernetzende Monomer unter Diallylphthalat, Allyl(meth)-acrylat, Divinylbenzol, (Poly)ethylenglykoldimethacrylat, Methylenbis-acrylamid, Polyallysaccharose und Polyallylpentaerythrit ausgewählt ist.

**18.** Zusammensetzung nach einem der vorhergehenden Ansprüche, **dadurch gekennzeichnet, daß** das anionische amphiphile Polymer, das mindestens eine Fettkette aufweist, in einer Menge von 0,03 bis 30 Gew.-%, bezogen auf die wasserfrei Zusammensetzung, vorliegt.

**19.** Zusammensetzung nach einem der vorhergehenden Ansprüche, **dadurch gekennzeichnet, daß** die Base unter den Alkalisilicaten, Alkaliphosphaten, Alkalicarbonaten, Erdalkalisilicaten, Erdalkaliphosphaten oder Erdalkalicarbonaten und insbesondere Alkalimetasilicaten ausgewählt ist.

**20.** Zusammensetzung nach einem der vorhergehenden Ansprüche, **dadurch gekennzeichnet, daß** das Peroxidsalz unter den Persulfaten, Percarbonaten und Perboraten von Ammonium oder Alkalimetallen und insbesondere Natriumpersulfat und Kaliumpersulfat ausgewählt ist.

**21.** Zusammensetzung nach Anspruch 20, **dadurch gekennzeichnet, daß** sie 30 bis 60 Gew.-% mindestens eines Peroxidsalzes enthält.

**22.** Zusammensetzung nach einem der vorhergehenden Ansprüche, **dadurch gekennzeichnet, daß** sie ferner Zusatzstoffe zum Bleichen enthält, die unter Mitteln zur Regulierung der Sauerstoffentwicklung, anionischen, nichtionischen, kationischen, amphoteren oder zwitterionischen grenzflächenaktiven Stoffen und ihren Gemischen, Mineralölen, pflanzlichen Ölen, Wachsen, Hilfsstoffen für die Granulierung, Bindemitteln, Füllstoffen, Trübungsmitteln, Farbmitteln, Maskierungsmitteln, Parfums und kationischen oder amphoteren substantiven Polymeren ausgewählt sind.

**23.** Zusammensetzung nach einem der vorhergehenden Ansprüche, **dadurch gekennzeichnet, daß** sie 0,01 bis 40 Gew.-% und vorzugsweise 0,1 bis 30 Gew.-% mindestens eines grenzflächenaktiven Stoffes enthält.

**24.** Zusammensetzung nach einem der vorhergehenden Ansprüche, **dadurch gekennzeichnet, daß** sie 0,03 bis 30 Gew.-% mindestens eines kationischen oder amphoteren substantiven Polymers enthält.

**25.** Zusammensetzung nach einem der vorhergehenden Ansprüche, **dadurch gekennzeichnet, daß** sie in Form eines Pulvers oder einer Suspension oder Dispersion von Pulver in einem wasserfreien flüssigen organischen Träger vorliegt.

**26.** Verwendung der wasserfreien Zusammensetzung zum Bleichen nach einem der vorhergehenden Ansprüche zur Herstellung einer gebrauchsfertigen Zusammensetzung zum Bleichen durch Zusatz einer wäßrigen Wasserstoffperoxid-Zubereitung.

**27.** Verfahren zum Bleichen von Keratinfasern und insbesondere menschlichen Haaren mit folgenden Schritten:

- die wasserfreie Zusammensetzung zum Bleichen nach einem der Ansprüche 1 bis 25 wird unmittelbar vor der Anwendung mit einer wäßrigen Wasserstoffperoxid-Zubereitung vermischt,
- das Gemisch wird auf den Bereich der zu blondierenden Keratinfasern aufgetragen,
- das Gemisch wird während einer Zeitspanne, die ausreichend ist, um die gewünschte Blondierung zu erzielen, einwirken gelassen, und
- das Gemisch zum Bleichen wird durch Spülen mit Wasser entfernt, worauf mit Haarwaschmittel gewaschen und getrocknet wird.

**28.** Vorrichtung mit mehreren Abteilungen oder "Kit" zum Bleichen von Keratinfasern und insbesondere menschlichen Haaren, wie dem Haar, **dadurch gekennzeichnet, daß** sie mindestens zwei Abteilungen aufweist, wobei eine Abteilung eine wasserfreie Zusammensetzung nach einem der Ansprüche 1 bis 25 und eine andere Abteilung eine wäßrige Wasserstoffperoxid-Zubereitung enthält.